# EUROPEAN PATENT APPLICATION

(11) **EP 2 554 185 A1**
(43) Date of publication of application: **06.02.2013**
(21) Application number: 11762237.3
(22) Date of filing: 29.03.2011
(51) Int. Cl.: A61K 47/44, A23D 9/00, A23L 1/00, A61K 47/14, B01J 13/04

(54) **COATING FAT COMPOSITION AND PARTICULATE COMPOSITION USING SAME**

(30) Priority: 04.11.2010 JP 2010247766; 29.03.2010 JP 2010075256
(71) Applicant: Kaneka Corporation, Osaka-shi, Osaka 530-8288 (JP)
(72) Inventor: KANAYA, Kento, Hyogo 676-8688 (JP); ABE, Masayuki, Hyogo 676-8688 (JP); SAKAKI, Akio, Hyogo 676-8688 (JP); SATO, Masao, Hyogo 676-8688 (JP)
(74) Representative: Vossius & Partner
(86) International application number: PCT/JP2011/001848
(87) International publication number: WO 2011/121989

(57) **Abstract**

Provided are a particulate composition which is a solid at room temperature, which has excellent oxidative stability, which encapsulates a large quantity of an enclosure such as a hydrophilic substance, which, when coating the enclosure, causes only minimal heat-induced damage to the enclosure, which is capable of rapidly releasing the coated enclosure when the composition reaches the intestines; and a fat composition capable of being used in the particulate composition. Disclosed are a particulate composition, wherein a hydrophilic substance is polydispersed in a matrix of a fat composition having a solid fat content at 25°C of 58% or more and a solid fat content at 37°C of 90% or less; and a coating fat composition containing 45% by weight or more of a triglyceride comprising at least both a saturated fatty acid having 6 to 12 carbon atoms and a saturated fatty acid having 14 or more carbon atoms as constituent fatty acids, wherein the proportion of the saturated fatty acid having 14 or more carbon atoms in the constituent fatty acids of the whole fat exceeds 50% by weight.

## Description

### Technical Field

The present invention relates to a particulate composition in which a hydrophilic substance is polydispersed in a matrix of a fat composition, and to a coating fat composition that is a solid at room temperature.

### Background Art

An S/O type or W/O type microcapsule can be employed for various applications such as foods, functional nutritive foods, specific health foods, medicines, cosmetics, feeds, and agrochemicals, by enclosing an useful component (core substance) in a predominately solid fat phase. For microcapsules to be used for such applications, there are demands not only for an increase in yield, a high content of a core substance, and a wide choice range of capsule particle diameter but also for control of a pattern of the release of a core substance from the viewpoint of DDS.

On the other hand, examples of the S/O type microcapsules heretofore known include microcapsules produced by the in-liquid drying process (Patent Document 1). Such microcapsules are difficult to use for food applications because organic solvents harmful to the human body, such as halogenated hydrocarbons or ethers, are used in their production. Moreover, the microcapsules produced by the in-liquid drying process can be used as controlled release microcapsules, but they are problematic in that physical fine pores appear easily in a capsule casing, a core substance easily leaks out of the casing or the like.

There has been proposed an S/O type microcapsule produced by preparing a fine W/O/W emulsion by membrane emulsification using a solid fat as a shell material, followed by freeze-drying (Patent Document 2), but it is difficult to enclose a high content of a core substance and there are problems, such as pressure loss or clogging during membrane emulsification and durability of membranes.

Moreover, there are known a W/O type or S/O type microcapsule in which a hydrophilic bioactive substance has been coated with tripalmitin and a method for its production by spray drying (Patent Document 3). In this document, the release characteristic of an enclosed substance has been revealed by immersing the microcapsule in a simulated intestinal fluid containing lipase. However, the microcapsule fails to exhibit a sufficient rate in releasing the enclosure in spite of the fact that it is a fine particle produced by spray-drying.

Moreover, since the melting point of the tripalmitin to be used is high, this method requires an operation of bringing the enclosure into contact with tripalmitin molten at a high temperature of 70°C or higher when dispersing the enclosure in a fat. For this reason, in the event that a material having low heat resistance is used for the enclosure, there is a problem that the deterioration or damage of the enclosure is caused, so that expected bioactive effects fail to be demonstrated sufficiently.

Incidentally, coating fats are used as base materials of microcapsules enclosing bioactive substances as described above and also are in use for coating of foods, and the like. On the other hand, in conventional coating of foods, for example, semi-solid fats have been used for coating snack foods (Patent Document 4), but there are problems, such as the deformation of a coating fat during storage and the sticking of coating fats.

Moreover, as to enteric properties, a coating fat is required to have a characteristic that it does not disintegrate in the stomach in oral ingestion, and it rapidly releases the enclosure on arrival at the intestines. However, it is hard to be said that a conventional coating fat sufficiently satisfies the above characteristic.
Patent Document 1: JP-A-2003-252751
Patent Document 2: JP 4038585
Patent Document 3: JP-A-2004-143084
Patent Document 4: JP-A-2003-61576

### DISCLOSURE OF THE INVENTION

### Problems to be Solved by the Invention

An object of the present invention is to provide a fine particulate composition which is a solid at room temperature, which has excellent oxidative stability, which encapsulates a large quantity of an enclosure such as a hydrophilic substance, which, when coating the enclosure, causes only minimal heat-induced damage to the enclosure, which has enteric properties to be capable of rapidly releasing the coated enclosure when the composition reaches the intestines, and which can be employed for a wide variety of applications such as foods and medicines; and a fat composition which can be used in the particulate composition.

### Means for Solving the Problems

The present inventors have investigated earnestly in order to solve the aforementioned problems of the present invention and, as a result, they have found that a particulate composition using a fat composition having a solid fat content within a specific range as a matrix can encapsulate a hydrophilic substance in a high content and also control the release of an enclosed hydrophilic substance in the intestinal tract, and therefore have completed a first invention.

That is, the first present invention relates to a particulate composition, wherein a hydrophilic substance is polydispersed in a matrix of a fat composition having a solid fat content at 25°C of 58% or more and a solid fat content at 37°C of 90% or less. A preferred embodiment thereof relates to a particulate composition, wherein the fat composition is composed of 95 to 60% by weight of a high-melting-point fathigh-melting-point fat and 5 to 40% by weight of a low-melting-point oil.

Moreover, the present inventors have investigated earnestly in order to solve a problem of a second present invention and, as a result, they have found that a fat composition having a specific saturated fatty acid composition as a constituent fatty acid can be processed at low temperatures because its melting point is not very high, but it can hold its solid state at room temperature and is excellent in oxidative stability and also it exhibits excellent enteric properties by coating an enclosure with the fat composition, and therefore have completed the second invention.

That is, the second present invention relates to a coating fat composition containing 45% by weight or more of a triglyceride having at least both a saturated fatty acid having 6 to 12 carbon atoms and a saturated fatty acid having 14 or more carbon atoms as constituent fatty acids, wherein the proportion of the saturated fatty acid having 14 or more carbon atoms in the constituent fatty acids of the whole fat exceeds 50% by weight. The coating fat composition can be used as the fat composition in the first present invention.

### Effects of the Invention

According to the present invention, there can be provided a fine particulate composition which is a solid at room temperature, which has excellent oxidative stability, which can include an enclosure such as a hydrophilic substance at a high content, and further which, when coating the enclosure, causes only minimal heat-induced damage to the enclosure because the coating can be performed at an operation temperature of, for example, 60°C or lower, at which coating using conventional coating fats is difficult to perform, and which can release the enclosure in the intestines efficiently without decomposing the enclosure in the stomach. Moreover, the present invention can be developed into a wide variety of application fields, not only to the fields of medicines and agrochemicals but also to the field of foods.

Furthermore, a coating fat composition capable of being used as a matrix base of the aforementioned particulate composition can also be provided. The coating fat composition can be developed into a wide variety of application fields because it is of high safety, for example, it can be used as an edible fat, and it can be applied easily not only to medicines in view of DDS, but also to the field of foods and functional foods.

### BRIEF DESCRIPTION OF THE DRAWINGS

Fig. 1 is a SEM photograph of a particulate composition produced in Example 1.
Fig. 2 is a SEM photograph of a particulate composition after a disintegration test performed in Example 1.

### PREFERRED MODE FOR CARRYING OUT THE INVENTION

Embodiments of the present invention will be described in detail below.

### (First present invention)

The particulate composition of the first present invention is a particulate composition, wherein a hydrophilic substance is polydispersed in a matrix of a fat composition having a solid fat content at 25°C of 58% or more and a solid fat content at 37°C of 90% or less.

A solid fat content (solid fat index) is generally a proportion accounted for by a fat component having been crystallized and solidified at a specific temperature in a fat; and in the present application, a fat composition having a solid fat content at 25°C of 58% or more and a solid fat content at 37°C of 90% or less is used as a matrix of the particulate composition. In the present application, the solid fat content is determined by heating a fat composition to be measured to or above its melting point to melt it completely, holding it at a prescribed temperature, that is, 25°C or 37°C, for one week, and then performing measurement by a known method. The known method for measuring the solid fat content is not particularly restricted, and any method may be used such as a method in which a dilatometer is used and an NMR method, which are disclosed in The JOCS Standard Methods for the Analysis of Fats, Oils and Related Materials (edited by Japan Oil Chemists' Society) or the like; in Examples disclosed below, the NMR method is adopted by which measurement can be performed more easily.

The fat composition to be used for the particulate composition of the present invention is not particularly restricted as far as its solid fat content at 25°C is 58% or more and its solid fat content at 37°C is 90% or less as described above. A fat composition having a solid fat content at 25°C of less than 58% cannot exhibit solidity at normal temperature (usually 25°C); even if such a fat composition is used, it is not possible to produce a particulate composition or, even if it is possible, a resulting particulate composition will be particles which are semi-solid and sticky at normal temperature and it will be very difficult to handle the particles due to the occurrence of sticking of the particles together or the like. From such a viewpoint, the fat composition preferably has a solid fat content at 25°C of 60% or more. If the solid fat content at 37°C exceeds 90%, the release rate of a hydrophilic substance in the intestines is low and therefore sufficient enteric properties cannot be achieved. From the ease of production, a fat composition having a solid fat content at 37°C of 50% or more and 90% or less is preferred, and a fat composition having a solid fat content at 37°C of 60% or more and 80% or less is more preferred. From the viewpoint of enteric properties, a fat composition having a solid fat content at 37°C of 50% or more and 85% or less is preferred, a fat composition having a solid fat content at 37°C of 50% or more and 75% or less is more preferred, and a fat composition having a solid fat content at 37°C of 50% or more and 70% or less is particularly preferred. On the other hand, in view of ease of handling, a fat composition having a solid fat content at 37°C of 60% or more and 90% or less is preferred. The fat composition to be used for the particulate composition of the present invention is a fat composition that has the above-described solid fat content and it is usually a fat composition that is a solid or exhibits solidity at normal temperature as described above.

The fat composition to be used for the particulate composition of the present invention is not particularly restricted if it is one having the above-described solid fat content. For example, naturally occurring fats or industrially produced fats can be used as they are or ones obtained by separating and/or curing such fats can be used. Moreover, a fat composition prepared by mixing two or more of the fats described above is also usable. Furthermore, a fat composition produced using a transesterification reaction can also be used. From the viewpoint of enteric properties, a fat composition produced using a transesterification reaction is preferred.

As the fat composition having the above-described solid fat content, the coating fat composition in the second present invention can be used, and the composition will be described below.

The fat composition having the above-described solid fat content can be prepared by mixing a high-melting-point fat having a melting point of 40°C or higher and a low-melting-point oil having a melting point of 355°C or lower in desired proportions. This is desirable in the application to food because the solid fat content in the fat composition can be freely controlled by simple procedures and it is possible to produce the fat composition of the present invention without using an organic solvent during its production. More preferable from the viewpoint of enteric properties is a mixture of a fat having a melting point of 45°C or higher which is a solid, disintegration-resistant, hard form at normal temperature as the high-melting-point fat and a liquid fat having a melting point of 25°C or less which is liquid at normal temperature as the low-melting-point oil. The terms "high-melting-point fat" and "low-melting-point oil" as used herein each mean a property which is exhibited by a whole mixture where two or more components are combined together as fats to be used.

The high-melting-point fat to be used for the present invention is not particularly restricted if it is a fat having a melting point of 40°C or higher. Examples of such a fat include animal and vegetable fats, such as palm oil, shea butter, sal butter, illipe butter, lard, and beef tallow; hydrogenated animal and vegetable oils, such as palm hardened oil, fully hardened palm oil, hardened rapeseed oil, fully hardened rapeseed oil, hardened soybean oil, hardened lard oil, and hardened fish oil; fractionated oils obtained by fractionally purifying high boiling fractions of animal and vegetable fats and their hydrogenated products, such as fractionated coconut oil, fractionated palm kernel oil, fractionated palm oil, fractionated cacao butter oil, fractionated shea butter oil, fractionated lard oil, fractionated hardened soybean oil, and fractionated hardened fish oil; saturated fatty acid triglycerides, such as tristearin, tripalmitin, and trilaurin; their partial glycerides, such as monoglycerides, diglycerides, and fatty acids; and edible waxes, such as cera flava, candelilla wax, and rice bran wax. As the high-melting-point fat in the present invention, these may be used alone or two or more of them may be used in combination. As such a high-melting-point fat, use of hardened oils, such as fractionated palm oil, hardened palm oil, hardened rapeseed oil, tristearin, and tripalmitin, and fractionated oils of high melting fractions is preferred from the viewpoint of easy availability and ease for performing melting and solidification by cooling. Moreover, use of fully hardened palm oil, fully hardened rapeseed oil, fractionated palm oil, tristearin, tripalmitin, and the like is preferred from the viewpoint of containing no trans-fatty acid. Among the above-mentioned high-melting-point fats, at least one member selected from the group consisting of fractionated palm oil, hardened palm oil, fully hardened palm oil, hardened rapeseed oil, fully hardened rapeseed oil, tristearin and tripalmitin is more preferred.

The low-melting-point oil to be used for the present invention is not particularly restricted if it is a fat having a melting point of 35°C or lower. Examples of such a fat include animal and vegetable fats, such as rapeseed oil, rice oil, peanut oil, olive oil, corn oil, soybean oil, perilla oil, cotton seed oil, sunflower oil, Oenothera Biennis oil, sesame oil, safflower oil, coconut oil, cacao butter, palm kernel oil, and fish oil; seaweed-derived fats, such as brown seaweed oil and sea tangle oil; microalgae-derived fats, such as Spirulina extracted oil; microorganism fats, such as yeast extracted oil and Mortierella extracted oil; fractionated oils obtained by fractionally purifying such low melting fractions, such as a low melting fraction of palm oil, a low melting fraction of fish oil, and a low melting fraction of shea butter; medium chain fatty acid triglycerides, such as tricaprylin and tricaprin; unsaturated fatty acid triglycerides, such as triolein and trilinol; their partial glycerides, such as monoglycerides and diglycerides; and fatty acids, such as oleic acid, linoleic oil, linolenic acid, arachidonic acid, eicosapentaenoic acid, and docosahexaenoic acid. As the low-melting-point oil in the present invention, these may be used alone or two or more of them may be used in combination. As such a low-melting-point oil, use of rapeseed oil, rice oil, corn oil, soybean oil, olive oil, sunflower oil, perilla oil, palm kernel oil, diglyceride, oleic acid, coconut oil, a low melting fraction of fish oil, medium chain fatty acid triglyceride, and the like is preferred from the viewpoint of easy availability. From the viewpoint of oxidative stability, use of rapeseed oil, corn oil, coconut oil, a low melting fraction of fish oil, medium chain fatty acid triglyceride, and on the like is preferred.

When the mixture of the high-melting-point fat and the low-melting-point oil is used as the fat composition to be used for the particulate composition of the present invention, their mixing ratio is not particularly restricted. However, a mixture of 95 to 60% by weight of the high-melting-point fat and 5 to 40% by weight of the low-melting-point oil is preferred. If the content of the high-melting-point fat is 95% by weight or more, resulting particles are hard and excellent in handleability, but release of a hydrophilic substance in the intestines tends to become slow. On the other hand, if the content of the high-melting-point fat is less than 60% by weight, since resulting particles will become soft, it may become difficult to handle the particles. In ease of production, preferred is a mixture of 85 to 70% by weight of the high-melting-point fat and 15 to 30% by weight of the low-melting-point oil, and more preferred from the viewpoint of enteric properties is a mixture of 80 to 60% by weight of the high-melting-point fat and 20 to 40% by weight of the low-melting-point oil. In ease of handling, more preferred is a mixture of 95 to 70% by weight of the high-melting-point fat and 5 to 30% by weight of the low-melting-point oil. Moreover, in ease of production, particularly preferred is a mixture of 80 to 70% by weight of the high-melting-point fat and 20 to 30% by weight of the low-melting-point oil, and particularly preferred from the viewpoint of enteric properties is a mixture of 70 to 60% by weight of the high-melting-point fat and 30 to 40% by weight of the low-melting-point oil. In ease of handling, particularly preferred is a mixture of 90 to 80% by weight of the high-melting-point fat and 10 to 20% by weight of the low-melting-point oil.

Moreover, the particulate composition of the present invention may contain a hydrophobic component other than the above-mentioned fats together with the fat composition to be used as a matrix. Use of bioactive substances or other useful components as such a hydrophobic component is preferable because it can cause resulting particulate compositions to contain not only hydrophilic substances but also hydrophobic components.

The hydrophobic substance to be used in this case may be selected according to an intended application as far as it is a substance capable of being dispersed or dissolved in the fat composition to be used. Examples of the hydrophobic substance include carotenoids, such as β-carotene, α-carotene, lycopene, lutein, astaxanthin, zeaxanthin, and fucoxanthin; hydrophobic flavonoids, such as quercetin, catechin, curcumin, and coumarin; low water-soluble anthocyanins, such as piperine, quercitrin, myricitrin, and naringin; alkaloids, such as capsaicin, capsiate, caffeine, berberine, and vincristine; hydrophobic vitamins, such as vitamin A, vitamin D, α-tocopherol, β-tocopherol, and vitamin K; hydrophobic lignans, such as sesamin, sesamolin, and sesaminol; hydrophobic coenzymes, such as coenzyme Q10. These substances may be used alone and two or more of them may be used in combination.

The hydrophilic substance to be enclosed in the particulate composition of the present invention may be selected according to an intended application as far as it is a substance that is soluble in water. Examples of the hydrophilic substance include proteins, peptides, amino acids, antibiotics, nucleic acids, organic acids, water-soluble vitamins, water-soluble polyphenols, water-soluble coenzymes, minerals, saccharides, terpene glycosides, and viable bacteria. The above-mentioned substances may be used in the form of derivatives or salts as far as they are hydrophilic. These substances may be used alone and two or more of them may be used in combination.

Examples of the proteins include enzymes, antibodies, antigens, and hormones. Specific examples thereof include proteases, amylases, cellulases, kinases, glucanases, pectinases, isomerases, lipases, pectinases, interferon, interleukin, BMP, immunoglobulin, serum albumin, and milk protein-derived ingredients (e.g., lactoferrin, lactoglobulin, lactoalbumin, and lactoperoxidase).

Examples of the peptides include luteinizing hormone releasing hormone (LH-RH), insulin, somatostatin, growth hormone, growth hormone releasing hormone (GH-RH), prolactin, erythropoietin, adrenocortical hormone, melanocyte stimulating hormone, thyrotropin releasing hormone (TRH), thyroid stimulating hormone, luteinizing hormone, follicle stimulating hormone, vasopressin, oxytocin, calcitonin, gastrin, secretin, pancreozymin, cholecystokinin, angiotensin, human placental lactogen, human chorionic gonadotropin, enkephalin, endorphin, kyotorphin, tuftsin, thymopoietin, thymosin, thymothymulin, thymic humoral factors, blood thymic factors, tumor necrosis factors, colony inducing factors, motilin, dynorphin, bombesin, neurotensin, cerulein, bradykinin, glutathione, imidazole dipeptides (e.g., carnosine, anserine, homoanserine, balenine, and aspartame), atrial natriuretic factors, nerve growth factors, cell growth factors, neurotrophic factors, peptides having endothelin antagonism, etc., and derivatives thereof, as well as fragments thereof and derivatives of such fragments.

Specific examples of the amino acids include glycine, alanine, valine, leucine, isoleucine, phenylalanine, tyrosine, tryptophan, serine, threonine, proline, hydroxyproline, cysteine, methionine, aspartic acid, glutamic acid, lysine, arginine, and histidine.

Examples of the antibiotics include β-lactam type, aminoglycoside type, tetracyclin type, chloramphenicol type, macrolide type, ketolide type, polyene macrolide type, glycopeptide type, nucleic acid type, and pyridonecarboxylic acid type antibiotics.

Specific examples of the nucleic acids include inosinic acid, guanylic acid, xanthylic acid, ATP, GTP, DNA, and RNA.

Specific examples of the organic acids include acetic acid, butyric acid, propionic acid, citric acid, succinic acid, fumaric acid, lactic acid, gluconic acid, malic acid, tartaric acid, and pyruvic acid.

Specific examples of the water-soluble vitamins include vitamin B1, vitamin B2, vitamin B6, vitamin B12, ascorbic acid, niacin, pantothenic acid, folic acid, lipoic acid, and biotin.

Specific examples of the water-soluble polyphenols include tea flavonoids, such as epigallocatechin gallate, epicatechin gallate, gallocatechin gallate, epigallocatechin, theaflavin, and theaflavin gallate; anthocyanins, such as nasunin, shisonin, and enin; flavonoids, such as naringin, hesperidin, and rutin; water-soluble lignans, such as sesaminol glucosides and pyredinol glycosides; and chlorogenic acid.

Examples of the water-soluble coenzymes include thiamine diphosphate, NADH, NAD, NADP, NADPH, FMN, FAD, coenzyme A, pyridoxal phosphate, and tetrahydrofolic acid.

Examples of the minerals include calcium, magnesium, iron, zinc, potassium, sodium, copper, vanadium, manganese, selenium, molybdenum, cobalt, and the like, as well as compounds to which such a mineral is bonded.

Examples of the saccharides include monosaccharides, disaccharides, oligosaccharides, sugar alcohols, and other polysaccharides. Specific examples of the monosaccharide include arabinose, xylose, ribose, glucose, fructose, galactose, mannose, sorbose, and rhamnose. Specific examples of the disaccharide include maltose, cellobiose, trehalose, lactose, and sucrose. Specific examples of the oligosaccharide include maltotriose, raffinose saccharide, and stachyose. Specific examples of the sugar alcohol include arabitol, xylitol, adonitol, mannitol, sorbitol, and dulcitol. Examples of the other polysaccharides include chitin, chitosan, agarose, heparin, hyaluronic acid, xyloglucan, starch, glycogen, pectin, chondroitin sulfate, heparan sulfate, and keratan sulfate.

Examples of the terpene glycosides include stevioside and glycyrrhizin.

The above-described viable bacteria are preferably ones suitable for mammals to oral consume; and examples thereof include lactobacillus, bifidobacteria, yeast, aspergillus, acetic acid bacteria, butyric acid bacteria, propionic acid bacteria, and saccharification bacteria.

Examples of the lactobacillus include Lactobacillus acidophilus, Lactobacillus bulgaricus, Lactobacillus brevis, Lactobacillus casei, Lactobacillus delbruekii, Lactobacillus fermrntii, Lactobacillus fermentum, Lactobacillus gasseri, Lactobacillus lactis, Lactobacillus leichmanii, Lactobacillus helveticus, Lactobacillus plantarum, Lactobacillus rhamnosus, Lactobacillus salivarius, Lactobacillus thermophilus, Lactobacillus pentosus, Lactococcus lactis, Lactococcus cremoris, Pediococcus acidilacticii, Pediococcus cerevisiae, Pediococcus pentosaceus, Leuconostoc mesenteroides, Leuconostoc lactis, Streptococcus lactis, Streptococcus cremoris, Streptococcus thermophilus, Streptococcus bulgaricus, Enterococcus faecalis, and Enterococcus faecium.

Examples of the bifidobacteria include Bifidobacterium adolescentis, Bifidobacterium angulatum, Bifidobacterium animalis, Bifidobacterium bifidum, Bifidobacterium breve, Bifidobacterium catenulatum, Bifidobacterium denticolens, Bifidobacterium dentium, Bifidobacterium gallicum, Bifidobacterium infantis, Bifidobacterium inopinatum, Bifidobacterium longum, and Bifidobacterium pseudocatenulatum.

Examples of the yeast include Saccharomyces boulardi, Saccharomyces cerevisiae, and Saccharomyces sake.

Examples of the aspergillus include Aspergillus oryzae, Aspergillus niger, and Aspergillus sojae.

Examples of the acetic acid bacteria include Acetobacter aceti, Acetobacter xylinum, and Acetobacter orientals. Examples of the butyric acid bacteria include Bacillus toyoi, Bacillus licheniformis, Clostridium butyricum, and Clostridium acetobutyricum. Examples of the propionic acid bacteria include Propionibacterium shermanii and Propionibacterium freudenreichii. Examples of the saccharification bacteria include Bacillus subtilis, Bacillus mesentericus, and Bacillus polyfermenticus.

In addition, examples of those suitable for mammals to oral consume include Bacillus coagulans and Bacillus pumilus. These viable bacteria may be used alone and also as a mixture of two or more of them.

In the particulate composition of the present invention, the weight ratio of the hydrophilic substance to the fat composition is preferably within the range of from 0.01/99.99 to 70/30 and more preferably within the range of from 1/90 to 40/60. In the event that the weight ratio of the hydrophilic substance to the fat composition is low, since the content of the hydrophilic substances in a resulting particulate composition becomes low, it becomes necessary, for example, to take a large amount of the particulate composition when a prescribed amount of the hydrophilic substance is orally administered. On the other hand, in the event that the weight ratio of the hydrophilic substance to the fat composition is excessively high, it is undesirable because the encapsulation yield of the hydrophilic substance will decrease, for example, the hydrophilic substance will leak to the outer aqueous phase in the production process.

The particulate composition of the present invention may further contain a surfactant, a thickener, a hydrophilic organic solvent, and the like. These may be ones used in the production of the particulate composition.

The particulate composition of the present invention is a particulate composition in which the hydrophilic substance is polydispersed in the fat composition having the above-described specific solid fat content, and it is a so-called S/O type or W/O type microcapsule.

The dispersion diameter of the hydrophilic substance in the particulate composition of the present invention is not particularly restricted, but it is preferably within the range of 0.01 to 50 µm, more preferably within the range of 0.01 to 20 µm, and most preferably within the range of 0.01 to 10 µm. The particulate composition of the present invention exhibits a particle-like shape at normal temperature and the average particle diameter of the particles is preferably 1 to 2000 µm, more preferably within the range of 10 to 1000 µm, and even more preferably within the range of 100 to 500 µm.

A method of producing the particulate composition of the present invention is not particularly restricted and methods of producing known S/O type microcapsules or W/O type microcapsules can be employed. For example, a solid particulate composition can be obtained by a method in which a fat composition, which is a matrix component, is adjusted to a temperature equal to or higher than its melting point, then a hydrophilic substance being in a solid or having been dissolved in water is mixed and dispersed in the fat composition, and then the resultant is subjected to liquid droplet dispersion in a gas phase or in a liquid phase and cooled to lower than the melting point of the fat composition. The S/O type microcapsule as used herein refers to a solid particle in which a hydrophilic solid substance is polydispersed in a matrix base composed of a fat composition, and it differs from an S/O suspension in which a solid substance is dispersed in a liquid oil phase and from an S/O/W emulsion in which the S/O suspension is suspended in an aqueous phase.

One preferred example of methods of making the dispersion diameter of a hydrophilic substance to be dispersed in a microcapsule as small as possible and making the content of the hydrophilic substance as high as possible is a method in which a mixture of a fat composition, which is a matrix component, and a hydrophilic substance to be enclosed or an aqueous solution of the hydrophilic substance is emulsified and dispersed at a temperature equal to or higher than the melting point of the fat composition, thereby preparing a W/O emulsion, then the moisture contained in the W/O emulsion is removed at a temperature that is equal to or higher than the melting point but is lower than the boiling point of the fat composition, thereby forming an S/O suspension, and then the S/O suspension is cooled to lower than the melting point of the fat composition while holding the suspension in a liquid droplet dispersion state in a gas phase or an aqueous phase to solidify the fat composition, thereby forming an S/O type solid particle. Alternatively, it is permitted to form an S/O type solid particle by dispersing a hydrophilic substance to be enclosed directly into a fat composition, which is a matrix component, at a temperature equal to or higher than the melting point of the fat composition, thereby preparing an S/o suspension, and then cooling the S/O suspension to lower than the melting point of the fat composition while holding the suspension in a liquid droplet dispersion state in a gas phase or an aqueous phase to solidify the fat composition; and it is also permitted to form a W/O type solid particle by emulsifying and dispersing a mixture of a fat composition, which is a matrix component, and a hydrophilic substance to be enclosed or an aqueous solution of the hydrophilic substance at a temperature equal to or higher than the melting point of the fat composition, thereby preparing a W/O emulsion, and then cooling the emulsion to lower than the melting point of the fat composition without removing moisture while holding the emulsion in a liquid droplet dispersion state to solidify the fat composition. Examples of a method of cooling the S/O suspension or the W/O emulsion to lower than the melting point of the fat composition while holding it in a liquid droplet dispersion state in a gas phase includes spray cooling methods. Examples of a method of cooling the S/O suspension or the W/O emulsion to lower than the melting point of the fat composition while holding it in a liquid droplet dispersion state in an aqueous phase include methods in which the S/O suspension or the W/O emulsion is added to an aqueous phase prepared separately (preferably, an aqueous phase containing a surfactant, a thickener, a hydrophilic organic solvent, and the like), thereby preparing an S/O/W emulsion or a W/O/W emulsion, and then cooling the resulting S/O/W emulsion or the W/O/W emulsion to lower than the melting point of the fat composition.

The present invention can provide a fine particulate composition which has a wide range of a particle diameter and in which a hydrophilic substance is enclosed in a high content. The particulate composition of the present invention has enteric properties due to use of a fat component decomposable by lipase as a matrix of the particulate composition and can be used as a formulation which allows a hydrophilic substance easily decomposable by the stomach, such as a protein, a peptide, or an enzyme, to be absorbed efficiently by the intestines without being decomposed in the stomach. Moreover, in the present invention, since the solubility or disintegrativity of a fat composition in the body, especially in the intestines, can be controlled through the adjustment of the solid fat content of the fat composition to be used as a matrix component to a desired range, it is possible to freely control the degree and timing of release of the enclosed hydrophilic substance from the particulate composition. The disintegrativity in the intestines of the particulate composition of the present invention can be set according to an intended purpose; for example, as for the release rate of a hydrophilic substance in an intestinal disintegration test described below, the release rate of the hydrophilic substance to be exhibited after treatment at 37°C for one hour is generally 20% or more, preferably 40% or more, more preferably 50% or more, even more preferably 60% or more, and particularly preferably 70% or more. Although it is needless to say that the upper limit of the release rate is 100%, a rate of about 95% is high enough for usual purposes.

### (Second present invention)

The coating fat composition of the second present invention contains, as a main component, a triglyceride including at least both a saturated fatty acid having 6 to 12 carbon atoms and a saturated fatty acid having 14 or more carbon atoms as constituent fatty acids. Triglycerides have three constituent fatty acids, and the triglyceride that is the main component of the coating fat composition of the present invention has at least one saturated fatty acid having 6 to 12 carbon atoms and at least one saturated fatty acid having 14 or more carbon atoms as constituent fatty acids. The remaining one constituent fatty acid in the above-mentioned triglyceride is not restricted; it may be either a saturated fatty acid or an unsaturated fatty acid, and the number of carbon atoms thereof may be chosen within an arbitrary range. From the viewpoint of controlling physical properties such as a melting point, preferred is one containing, as a main component, a triglyceride composed only of saturated fatty acid(s) having 6 to 12 carbon atoms and saturated fatty acid(s) having 14 or more carbon atoms as constituent fatty acids (namely, a triglyceride containing one saturated fatty acid having 6 to 12 carbon atoms and two saturated fatty acids having 14 or more carbon atoms as constituent fatty acids or a triglyceride containing two saturated fatty acids having 6 to 12 carbon atoms and one saturated fatty acid having 14 or more carbon atoms as constituent fatty acids). The term "main component" as used herein means that the content thereof in the fat composition accounts for at least 45% by weight, preferably 50% by weight or more, more preferably 60% by weight or more, and even more preferably 70% by weight or more. If the content of the aforementioned triglyceride in the coating fat composition of the present invention is less than 45% by weight, the enclosure coated with the coating fat composition of the present invention may be released at a low rate in the intestines, so that sufficient enteric properties may not be achieved.

Furthermore, the coating fat composition of the present invention is characterized in that as for the constituent fatty acid proportion in the whole fat contained in the composition, the proportion accounted for by the saturated fatty acid having 14 or more carbon atoms exceeds to by weight. If that proportion is 50% by weight or less, there is a tendency that the coating fat composition becomes liquid or semi-solid at room temperature, and therefore it is impossible to form a particulate composition or a resulting particulate composition is so soft that it is difficult to handle the composition or it has poor flowability.

That is, the coating fat composition of the present invention is a coating fat composition containing 45% by weight or more of a triglyceride including at least both a saturated fatty acid having 6 to 12 carbon atoms and a saturated fatty acid having 14 or more carbon atoms as constituent fatty acids, wherein the proportion of the saturated fatty acid having 14 or more carbon atoms in the constituent fatty acids of the whole fat exceeds 50% by weight. From the viewpoint of easy availability of raw materials, the coating fat composition of the present invention is preferably one containing, as a main component, a triglyceride including at least both a saturated fatty acid having 8 to 12 carbon atoms and a saturated fatty acid having 16 or more carbon atoms as constituent fatty acids, and more preferably one containing, as a main component, a triglyceride including at least both a saturated fatty acid having 8 to 12 carbon atoms and a saturated fatty acid having 16 or 18 carbon atoms as constituent fatty acids. The content of the triglyceride including at least both a saturated fatty acid having 8 to 12 carbon atoms and a saturated fatty acid having 16 or 18 carbon atoms in the coating fat composition of the present invention is preferably 50% by weight or more, more preferably 60% by weight or more, and even more preferably 70% by weight or more. Although the upper limit of the content of the triglyceride is not particularly restricted, it is preferably 90% by weight or less from the viewpoint of enteric properties.

The coating fat composition of the present invention is not be particularly restricted if it is one having the aforementioned constituent fatty acid composition and can be obtained by fractionating and/or hardening naturally occurring fats or industrially produced fats or mixing such fats so that the aforementioned constituent fatty acid composition may be achieved, but preferably it is produced by using a transesterification reaction because a specific triglyceride as a main component can be obtained easily. The transesterification reaction as used herein can be performed by using a fat, a fatty acid, a fatty acid ester, and the like as raw materials. Specific examples thereof include transesterification between two or more types of fats, transesterification between one or more types of fats and one or more types of fatty acids, a transesterification reaction between one or more types of fats and one or more types of fatty acid esters, and a transesterification reaction between one or more types of fats, one or more types of fatty acids, and one or more types of fatty acid esters, but any one of them may be employable. The fat to be used as a raw material of the transesterification reaction is not particularly restricted, and examples thereof include animal and vegetable fats, such as coconut oil, palm oil, palm kernel oil, cacao butter, shea butter, sal butter, illipe butter, lard, beef tallow, rapeseed oil, rice oil, peanut oil, olive oil, corn oil, soybean oil, perilla oil, cotton seed oil, sunflower oil, Oenothera Biennis oil, sesame oil, safflower oil, palm kernel oil, and fish oil; hydrogenated animal and vegetable oils, such as hardened palm oil, hardened rapeseed oil, hardened soybean oil, hardened lard, hardened fish oil, fully hardened palm oil, and fully hardened rapeseed oil; fractionated oils obtained by fractionating these, such as fractionated coconut oil, fractionated palm kernel oil, fractionated palm oil, fractionated cacao butter oil, fractionated shea butter oil, fractionated lard oil, fractionated hardened soybean oil, and fractionated hardened fish oil; saturated fatty acid triglycerides, such as tristearin, tripalmitin, and trilaurin; medium chain fatty acid triglycerides, such as tricaprylin and tricaprin; and unsaturated fatty acid triglycerides, such as triolein and trilinol, and their partial glycerides, such as monoglycerides and diglycerides. Further, examples of the fatty acid or fatty acid ester to be used as a raw material of the transesterification reaction include, but are not limited to, fatty acids, such as caprylic acid, capric acid, lauric acid, myristic acid, palmitic acid, stearic acid, oleic acid, linoleic oil, linolenic acid, arachidonic acid, eicosapentaenoic acid, and docosahexaenoic acid; and fatty acid esters, such as methyl esters or ethyl esters thereof.

The transesterification that can be performed in the present invention may be either a chemical transesterification reaction using an alkaline catalyst or the like or an enzymatic transesterification using an enzyme or a microorganism. There may or may not be a difference in composition between the fatty acid of the 1- or 3-position and the fatty acid of the 2-position of the resulting transesterified fat.

The catalyst capable of being used in performing the chemical transesterification reaction in the present invention is not particularly restricted as far as it is a catalyst generally known as a transesterification catalyst. Specific examples thereof include alkali metals, such as lithium, sodium, and potassium; alkaline earth metals, such as magnesium, calcium, and barium; metals such as zinc, cadmium, titanium, tin, antimony, lead, manganese, cobalt, and nickel; and acetates, carbonates, borates, oxides, hydroxides, hydrides, or alcoholate, such as methylate, thereof. In the present invention, the chemical transesterification reaction can be performed by, for example, fully drying raw materials such as a fat, a fatty acid, and a fatty acid ester, adding the aforementioned catalyst to the raw materials in an amount of 0.1 to 1% by mass, and then stirring under reduced pressure at 80 to 120°C for 0.5 to 1 hour in accordance with a routine procedure. After the completion of the transesterification reaction, the catalyst is washed away with water, and then decolorization and deodorization treatments may be performed which are performed in common purification steps for edible oils.

Although the enzyme or microorganism usable in performing the enzymatic transesterification reaction in the present invention is not particularly restricted as far as it is an enzyme generally known to be usable for a transesterification reaction, lipase or a microorganism that produces lipase is usually used. As to lipase to be used, any of a lipase solution, a lipase powder, and immobilized lipase in which a lipase powder is immobilized to a carrier such as cerite or an ion exchange resin may be used. Examples of the lipase usable for the enzymatic transesterification reaction in the present invention include lipase derived from genus Alcaligenes (e.g., lipase QLM and lipase PL produced by Meito Sangyo Co., Ltd., etc.), lipase derived from genus Candida (e.g., lipase OF produced by Meito Sangyo Co., Ltd., etc.), and immobilized lipase derived from Rhizomucor miehei (e.g., Lipozime TLIM and Lipozyme RMIM produced by Novozymes, etc.). Moreover, microorganisms which are sources of such lipase can be used as a catalyst for the enzymatic transesterification reaction directly in the form of dried fungus or an immobilized fungus. In the present invention, the enzymatic transesterification may be performed by, for example, adding an enzyme such as the aforementioned lipase to the aforementioned raw materials in an amount of 0.02 to 10% by mass, and preferably 0.04 to 5% by mass, while stirring the mixture at 40 to 85°C, and preferably 40 to 80°C for 0.5 to 48 hours, and preferably 0.5 to 24 hours. After the completion of the transesterification reaction, the enzyme such as a lipase powder or immobilized lipase, or fungus is removed by filtration or the like, and fatty acids or fatty acid esters are removed if necessary in the event that they are present after the reaction, and thereafter decolorization and deodorization treatments may be performed which are performed in common purification steps for edible oils.

Moreover, in the present invention, in order to adjust constituent fatty acid composition or physical properties such as melting point and solid fat content after the transesterification reaction, it is possible to further fractionate or crystallize the resulting transesterified fat or mix the fat with other fats or edible waxes such as cera lava, candelilla wax, and rice bran wax. As the other fats to be mixed in this case, fats such as those described above can be used.

The enclosure that can be coated with the coating fat composition of the present invention is not particularly restricted; bioactive substances or other effective ingredients may be enclosed directly or powders or tablets containing bioactive substances or effective ingredients, or the like may be enclosed. Moreover, foods and food base materials may also be coated. The bioactive substances or the other effective ingredients may be any of hydrophilic substances and hydrophobic substances, or may be both, and can be selected appropriately according to an intended application.

Examples of the hydrophilic substances and the hydrophobic substances that can be coated with the coating fat composition of the second present invention include the hydrophilic substances and the hydrophobic substances that are mentioned above for the first present invention.

The above-mentioned hydrophobic substance can be added either in a form in which it is enclosed as a mixture with other ingredients within a coating fat composition without being mixed completely with the coating fat composition or in a form in which it exists together with a coating fat composition while being completely or partially mixed therewith. For example, there may be employed a form in which the surface of a particulate composition, tablet or the like enclosing the hydrophobic substance is coated with the coating fat composition of the present invention. Moreover, a material prepared by dissolving the hydrophobic substance in the coating fat composition in advance also can be used as a base material for microcapsules or as a coating fat.

In the present invention, the form of coating the enclosure as described above with the coating fat composition of the present invention is not particularly restricted, and examples thereof include, in addition to a form of an S/O type microcapsule and a form of a W/O type microcapsule, forms of coated particles and coated tablets. Among these, the coating fat composition of the present invention is very suitable as the matrix base of an S/O type microcapsule as described above.

In the present invention, a method of producing an S/O type microcapsule using the coating fat composition of the present invention is not particularly restricted; however, the above-described methods are preferred, and specific examples thereof include production methods by a liquid phase process in which an S/O suspension in which a hydrophilic substance is polydispersed in a coating fat composition molten at equal to or higher than the melting point of the coating fat composition is prepared in advance, then the suspension is suspended in an aqueous phase to form liquid droplets, and cooling the droplets to lower than the melting point of the coating fat composition, thereby obtaining solid particles, and by a gas phase process in which the S/O suspension is directly sprayed and cooled to cool liquid droplets of the S/O suspension to lower than the melting point of the fat composition, thereby solidifying the fat composition.

In this case, a method of preparing the S/O suspension is not particularly restricted as far as it is a method capable of dispersing the hydrophilic substance uniformly in a molten coating fat composition; and for example, an S/O suspension can be obtained by mixing an aqueous solution containing a hydrophilic substance dissolved therein and a surfactant into a molten coating fat composition, followed by emulsification with a homogenizer or the like, thereby preparing a W/O emulsion, and then removing only moisture at high temperature under reduced pressure. On the other hand, as for materials low in heat resistance such as those suffer from deterioration, extinction, etc. at high temperature like proteins, peptides, and viable bacteria, an S/O suspension can be obtained by directly adding a hydrophilic substance and a surfactant that aids dispersion into a molten coating fat composition, and then fully dispersing them with a stirrer, a homogenizer, or the like.

In the event that an S/O type microcapsule is produced using the coating fat composition of the present invention, the weight ratio of a hydrophilic substance as an enclosure to the coating fat composition is preferably within the range of from 0.01/99.99 to 70/30, and more preferably within the range of from 1/90 to 40/60. In the event that the weight ratio of the hydrophilic substance to the coating fat composition is low, since the content of the hydrophilic substance in a resulting S/O type microcapsule becomes low, it becomes necessary, for example, to take a large amount of microcapsules when orally administering a prescribed amount of the hydrophilic substance. On the other hand, if the weight ratio of the hydrophilic substance to the coating fat composition is excessively high, it is difficult to obtain particles which are spherical and excellent in flowability and because of insufficient coating of an enclosed substance, most of the enclosed substance is released in the stomach before microcapsules arrive at the intestines when orally taken, so that an enteric effect will fail to be demonstrated sufficiently. For example, when proteins, viable bacteria, or the like with low resistance to stomach acid are used as the enclosed substance, their deterioration or extinction is caused, so that an expected bioactive effect may not be demonstrated.

On the other hand, in obtaining a coated particle or coated tablet in which the surface of a particulate composition or tablet is coated with the coating fat composition of the present invention, a coating method is not particularly restricted and, for example, it can be produced by spraying a molten coating fat composition to the surface of a particulate composition or tablet by using a pan coating device, fluidized bed granulation, or a coating device, thereby forming a film layer.

The coating fat composition of the present invention keeps a solid at room temperature and exhibits excellent intestinal disintegrativity even though it is easy to handle formulations or the like after coating therewith. For this reason, S/O type microcapsules, coated particles and coated tablets prepared using the coating fat composition of the present invention are excellent as enteric formulations which protect their enclosure from stomach acid in the stomach and release the enclosure rapidly after their arrival at the intestines.

### EXAMPLES

The present invention will be described more specifically below with reference to examples, but the invention is not limited thereto. In the examples, "'part(s)" and "%" mean "part(s) by weight" and "% by weight," respectively.

### (First present invention)

### (Measurement of solid fat content)

A fat composition to be measured was heated to equal to or higher than its melting point, thereby being melted completely. Then, it was held at prescribed temperature (25°C or 37°C) for one week, and thereafter a solid fat content at the prescribed temperature was measured in accordance with The JOCS Standard Methods for the Analysis of Fats, Oils and Related Materials (edited by Japan Oil Chemists' Society), 2.2.9-2003 by using a magnetic nuclear resonance instrument MINISPEC mq20 (manufactured by Bruker Optics Inc).

### (Intestinal disintegration test)

In a disintegration test of a particulate composition, evaluation was performed using a simulated intestinal fluid containing bile and lipase. The simulated intestinal fluid was prepared by dissolving a bile powder (produced by Wako Pure Chemical Industries, Ltd.) and lipase derived from porcine pancreas (produced by Sigma) in a Disintegration Test Fluid 2 of pH 6.8 (produced by Kanto Chemical Co., Inc.) with concentrations of 0.5% by weight, respectively. A particulate composition enclosing Food Red No.102 (produced by Wako Pure Chemical Industries, Ltd.) as a hydrophilic substance was added to the simulated intestinal fluid and shaken at 37°C for one hour, and then a 50% trichloroacetic acid (produced by Wako Pure Chemical Industries, Ltd.) aqueous solution was added and then subjected to centrifugal separation (MX-200, manufactured by Tomy Seiko Co, Ltd.) at 37°C, 12000 rpm for 5 minutes. By the measurement of absorption at 510 nm of the supernatant solution after the centrifugation by using a spectrophotometer (U-2000A Type, manufactured by Hitachi High-Technologies Corporation), the concentration of the hydrophilic substance in the supernatant solution was calculated. Assuming that the release rate achieved at the complete release of the hydrophilic substance contained in the particulate composition was considered to be 100%, the release rate of the hydrophilic substance achieved when treated at 37°C for one hour with the simulated intestinal fluid was calculated.

### (Measurement of average particle diameter of particulate composition)

Measurement was performed by using a particle diameter measurement device (LA-950, manufactured by HORIBA, Ltd.).

### (Content of hydrophilic substance in particulate composition)

The particulate composition obtained was liquefied by being heated to temperature equal to or higher than the melting point of the solid fat used, and then it was mixed with water, so that the hydrophilic substance encapsulated in the particulate composition was extracted into the aqueous phase. The concentration of the hydrophilic substance extracted into the aqueous phase was measured by HPLC and then the net content of the hydrophilic substance in the particulate composition was calculated.

### (Encapsulation yield of hydrophilic substance into particulate composition)

An encapsulation yield was calculated from the weight of the hydrophilic substance used during production and the content of the hydrophilic substance in the particulate composition calculated by the above-described method.

### (Production Example 1: Preparation of fish oil low melting fraction)

One hundred parts of fish oil deacidfied and bleached by a routine procedure was melted at 40°C and then cooled to 10°C under gentle agitation and further held at 10°C for 12 hours, so that crystals were precipitated. Subsequently, the resulting crystals were collected by suction filtration, whereby a fish oil low melting fraction that was liquid at 25°C was obtained.

### (Production Example 2: preparation of fractionated palm oil)

Two hundred parts of hexane was added to 100 parts of hardened palm part, which was then dissolved completely at 45°C and thereafter was held at 20°C for 20 hours, whereby a high melting fraction was precipitated. Subsequently, the resulting high melting fraction was collected by suction filtration and hexane was evaporated with an evaporator, followed by steam distillation at 250°C, whereby fractionated palm oil (melting point 52°C) was obtained.

### (Example 1)

Ninety grams of fully hardened palm oil (product name "RHPL", produced by Taiyo Yushi Corporation, melting point 57°C) and 10 g of medium chain fatty acid triglyceride (product name "Actor M2", produced by Riken Vitamin Co., Ltd., melting point -12°C) were mixed under heating, affording a fat composition. The solid fat content of the resulting fat composition was measured by the above-described method.

To an oily component composed of 20 g of a fat composition having been melted by being heated to a temperature of 70°C in advance and 1.0 g of tetraglycerol condensed ricinoleate (product name "POEM PR-100", produced by Riken Vitamin Co., Ltd.), 10 mL of a 10% by weight Food Red No.102 aqueous solution was added, followed by performing emulsification and dispersion with a homogenizer (T.K. Homomixer MARK II 20 type, manufactured by PRIMIX Corporation), whereby a W/O emulsion was prepared. Subsequently, the W/O emulsion was subjected to moisture removal while being stirred at a temperature of 70°C for 30 minutes under a reduced pressure condition of 13 kPa, whereby an S/O suspension was obtained. The S/O suspension was added to 200 mL of an aqueous solution having been heated to 70°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and the mixture was stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby a particulate composition was obtained. An intestinal disintegration test of the resulting particulate composition was performed by the above-mentioned method. The solid fat content of the fat composition used and the results of the intestinal disintegration test of the particulate composition obtained were shown in Table 1.

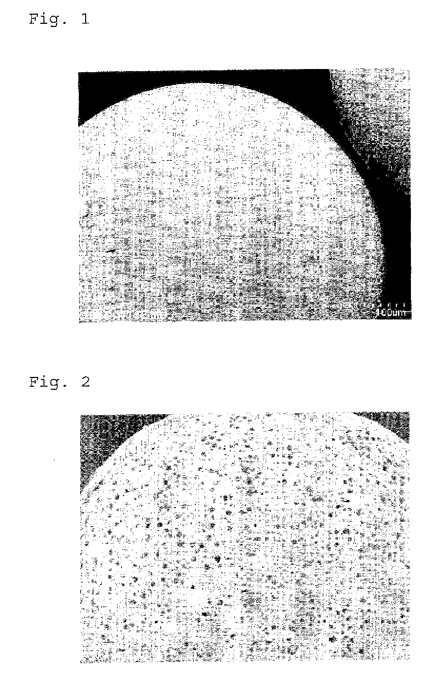

The average particle diameter of the resulting particulate composition was 351 µm, and the encapsulation yield of Food Red No.102 into the particulate composition in this example was 93.5%. Moreover, when the resulting particulate composition was observed with a scanning electron microscope (S-4800, manufactured by Hitachi, Ltd.; hereinafter SEM), a particle shape having a smooth surface structure as shown in Fig. 1 was observed. Furthermore, when the surface structure after the disintegration test of the particulate composition was observed by SEM, fine pores illustrated in Fig. 2 were observed. It is conceivable that Food Red No.102, which is the enclosed substance, was released through the pores.

### (Example 2)

A particulate composition was obtained in the same manner as in Example 1 except that a material prepared by heat-mixing 70 g of the fractionated palm oil prepared in Production Example 2, 20 g of fully hardened rapeseed oil (produced by Yokozeki Oil & Fat Industries Co., Ltd., melting point 67°C), and 10 g of medium chain fatty acid-containing triglyceride (product name "Actor M1", produced by Riken Vitamin Co., Ltd., melting point -6°C) was used as a fat composition. The solid fat content of the fat composition used and the results of the intestinal disintegration test of the particulate composition obtained were shown in Table 1.

### (Example 3)

A particulate composition was obtained in the same manner as in Example 1 except that a material prepared by heat-mixing 95 g of the fractionated palm oil prepared in Production Example 2 and 5 g of the fish oil low melting fraction prepared in Production Example 1 was used as a fat composition. The solid fat content of the fat composition used and the results of the intestinal disintegration test of the particulate composition obtained were shown in Table 1.

### (Example 4)

A particulate composition was obtained in the same manner as in Example 1 except that a material prepared by heat-mixing 80 g of fully hardened rapeseed oil (produced by Yokozeki Oil & Fat Industries Co., Ltd.) and 20 g of medium chain fatty acid triglyceride (product name "Actor M2", produced by Riken Vitamin Co., Ltd.) was used as a fat composition. The solid fat content of the fat composition used and the results of the intestinal disintegration test of the particulate composition obtained were shown in Table 1.

### (Example 5)

A particulate composition was obtained in the same manner as in Example 1 except that a material prepared by heat-mixing 60 g of fully hardened rapeseed oil (produced by Yokozeki Oil & Fat Industries Co., Ltd.) and 40 g of medium chain fatty acid triglyceride (product name "Actor M2", produced by Riken Vitamin Co., Ltd.) was used as a fat composition. The solid fat content of the fat composition used and the results of the intestinal disintegration test of the particulate composition obtained were shown in Table 1.

### (Example 6)

A particulate composition was obtained in the same manner as in Example 1 except that a material prepared by heat-mixing 85 g of fully hardened rapeseed oil (produced by Yokozeki Oil & Fat Industries Co., Ltd.) and 15 g of olive oil (produced by The Nisshin OilliO Group, Ltd.) was used as a fat composition. The solid fat content of the fat composition used and the results of the intestinal disintegration test of the particulate composition obtained were shown in Table 1.

### (Example 7)

A particulate composition was obtained in the same manner as in Example 1 except that a material prepared by heat-mixing 85 g of fully hardened rapeseed oil (produced by Yokozeki Oil & Fat Industries Co., Ltd.) and 15 g of sunflower oil (produced by Showa Sangyo Co., Ltd.) was used as a fat composition. The solid fat content of the fat composition used and the results of the intestinal disintegration test of the particulate composition obtained were shown in Table 1.

### (Example 8)

A particulate composition was obtained in the same manner as in Example 1 except that a material prepared by heat-mixing 85 g of fully hardened rapeseed oil (produced by Yokozeki Oil & Fat Industries Co., Ltd.) and 15 g of perilla oil (produced by Ohta Oil Mill Co., Ltd.) was used as a fat composition. The solid fat content of the fat composition used and the results of the intestinal disintegration test of the particulate composition obtained were shown in Table 1.

### (Example 9)

A particulate composition was obtained in the same manner as in Example 1 except that a material prepared by heat-mixing 85 g of fully hardened rapeseed oil (produced by Yokozeki Oil & Fat Industries Co., Ltd.) and 15 g of diglyceride (product name "Econa", produced by Kao Corporation) was used as a fat composition. The solid fat content of the fat composition used and the results of the intestinal disintegration test of the particulate composition obtained were shown in Table 1.

### (Example 10)

A particulate composition was obtained in the same manner as in Example 1 except that a material prepared by heat-mixing 85 g of fully hardened rapeseed oil (produced by Yokozeki Oil & Fat Industries Co., Ltd.) and 15 g of oleic acid (produced by Wako Pure Chemical Industries, Ltd.) was used as a fat composition. The solid fat content of the fat composition used and the results of the intestinal disintegration test of the particulate composition obtained were shown in Table 1.

### (Example 11)

A particulate composition was obtained in the same manner as in Example 1 except that a material prepared by heat-mixing 85 g of trilaurin (produced by Wako Pure Chemical Industries, Ltd., melting point 45°C) and 15 g of palm kernel oil (produced by Kaneka Corporation, melting point 27°C) was used as a fat composition. The solid fat content of the fat composition used and the results of the intestinal disintegration test of the particulate composition obtained were shown in Table 1.

### (Example 12)

A particulate composition was obtained in the same manner as in Example 1 except that a material prepared by heat-mixing 85 g of fully hardened rapeseed oil (produced by Yokozeki Oil & Fat Industries Co., Ltd.) and 15 g of rapeseed oil (produced by Kaneka Corporation) was used as a fat composition. The solid fat content of the fat composition used and the results of the intestinal disintegration test of the particulate composition obtained were shown in Table 1.

### (Example 13)

A fat composition was obtained by heat-mixing 85 g of fully hardened rapeseed oil (produced by Yokozeki Oil & Fat Industries Co., Ltd.) and 15 g of rapeseed oil (produced by Kaneka Corporation). To an oily component composed of 20 g of a fat composition having been melted by being heated to a temperature of 70°C in advance and 1.0 g of tetraglycerol condensed ricinoleate (product name "POEM PR-100", produced by Riken Vitamin Co., Ltd.), 5 mL of an aqueous solution containing 30% by weight of anserine (produced by Yaizu Suisankagaku Industry Co., Ltd.) was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 70°C for 30 minutes under a reduced pressure condition of 13 kPa, whereby an S/O suspension was obtained. The S/O suspension was added to 200 mL of an aqueous solution having been heated to 70°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and the mixture was stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby a particulate composition was obtained. The average particle diameter of the resulting particulate composition was 285 µm, and the encapsulation yield of anserine into the particulate composition in this example was 89.0%.

### (Example 14)

A particulate composition was obtained in the same manner as in Example 1 except that a material prepared by heat-mixing 90 g of the fractionated palm oil prepared in Production Example 2 and medium chain fatty acid triglyceride was used as a fat composition. The solid fat content of the fat composition used and the results of the intestinal disintegration test of the particulate composition obtained were shown in Table 1.

### (Example 15)

A fat composition was obtained by heat-mixing 90 g of the fractionated palm oil prepared in Production Example 2 and 10 g of medium chain fatty acid triglyceride (product name "Actor M2", produced by Riken Vitamin Co., Ltd.). Then, 5 g of lactoferrin (produced by Wako Pure Chemical Industries, Ltd.) and 1.5 g of sucrose erucate (product name "ER-290", produced by Mitsubishi-Kagaku Foods Corporation) were added to 200 mL of ethanol and dispersed with a homogenizer while being heated to 40°C, whereby a mixed liquid was prepared. Ethanol was removed by stirring the mixed liquid at 45°C for 30 minutes under a reduced pressure condition of 13 kPa, whereby a complex of lactoferrin and sucrose erucate was obtained. To 18 g of a fat composition having been melted by heating to a temperature of 55°C in advance, the complex obtained above was added and then dispersed with a homogenizer, whereby an S/O suspension containing the lactoferrin complex dispersed therein was obtained. Subsequently, the S/O suspension was added to 300 mL of an aqueous solution having been heated to 55°C in advance and containing 0.5% by weight of gum arabic (product name "Gum Arabic SD", produced by San-Ei Gen F.F.I., Inc.) and 0.01% by weight of decaglycerol monolaurate (product name "ML-750", produced by Sakamoto Yakuhin Kogyo Co., Ltd.) and stirred with a disk turbine blade for 10 minutes, whereby an S/O/W emulsion was prepared. Thereafter, the S/O/W emulsion was added in one portion to 300 mL of an aqueous solution having been cooled to 15°C in advance and containing 0.5% by weight of gum arabic and 0.01% by weight of decaglycerol monolaurate to be cooled rapidly, followed by suction filtration and vacuum drying, whereby a particulate composition was obtained.

The resulting particulate composition did not disintegrate even though it was added to a simulated gastric liquid, and when the particulate composition was removed from the simulated gastric liquid 120 minutes after addition and then the lactoferrin enclosed in the particulate composition was analyzed, it was confirmed that the lactoferrin in the particulate composition was present without being decomposed. That is, it was demonstrated that the lactoferrin polydispersed in the particulate composition of this example was provided with digestive enzyme resistance in the stomach.

### (Example 16)

A particulate composition was obtained in the same manner as in Example 1 except that a material prepared by heat-mixing 18 g of the fractionated palm oil prepared in Production Example 2, 2 g of medium chain fatty acid triglyceride (product name "Actor M2", produced by Riken Vitamin Co., Ltd.) and 10 g of Coenzyme Q10 (produced by Kaneka Corporation) was used as a fat composition. The solid fat content of the fat composition used and the results of the intestinal disintegration test of the particulate composition obtained were shown in Table 1.

### (Example 17)

A fat composition was obtained by heat-mixing 18 g of the fractionated palm oil prepared in Production Example 2, 2 g of medium chain fatty acid triglyceride (product name "Actor M2", produced by Riken Vitamin Co., Ltd.) and 10 g of Coenzyme Q10 (produced by Kaneka Corporation). Then, 4.2 g of lactoferrin (produced by Wako Pure Chemical Industries, Ltd.) and 0.9 g of sucrose erucate (product name "ER-290", produced by Mitsubishi-Kagaku Foods Corporation) were added to 200 mL of ethanol and dispersed with a homogenizer while being heated to 40°C, whereby a mixed liquid was prepared. Ethanol was removed by stirring the mixed liquid at 45°C for 30 minutes under a reduced pressure condition of 13 kPa, whereby a complex of lactoferrin and sucrose erucate was obtained. To 15 g of a fat composition having been melted by heating to a temperature of 58°C in advance, the complex was added and then dispersed with a homogenizer, whereby an S/O suspension containing the lactoferrin complex dispersed therein was obtained. Subsequently, the S/O suspension was added to 300 mL of an aqueous solution having been heated to 55°C in advance and containing 0.5% by weight of gum arabic (product name "Gum Arabic SD", produced by San-Ei Gen F.F.I., Inc.) and 0.01% by weight of decaglycerol monolaurate (product name "ML-750", produced by Sakamoto Yakuhin Kogyo Co., Ltd.) and stirred with a disk turbine blade for 10 minutes, whereby an S/O/W emulsion was prepared. Thereafter, the S/O/W emulsion was added in one portion to 300 mL of an aqueous solution having been cooled to 15°C in advance and containing 0.5 % by weight of gum arabic and 0.01% by weight of decaglycerol monolaurate to be cooled rapidly, followed by suction filtration and vacuum drying, whereby a particulate composition was obtained. The average particle diameter of the resulting particulate composition was 374 µm, and the encapsulation yield of lactoferrin into the particulate composition in this example was 94.8%.

### (Comparative Example 1)

To an oily component composed of 20 g of tripalmitin (produced by Wako Pure Chemical Industries, Ltd.) having been melted by being heated to a temperature of 80°C in advance and 1.0 g of tetraglycerol condensed ricinoleate (product name "POEM PR-100", produced by Riken Vitamin Co., Ltd.), 10 mL of an aqueous solution containing 10% by weight of Food Red No.102 was added, followed by performing emulsification and dispersion with a homogenizer, whereby a W/O emulsion was prepared. Subsequently, the W/O emulsion was subjected to moisture removal while being stirred for 20 minutes at a temperature of 80°C under a reduced pressure condition of 13 kPa, whereby an S/O suspension was obtained. The S/O suspension was added to 300 mL of an aqueous solution having been heated to 70°C in advance and containing 0.5% by weight of gum arabic (product name "Gum Arabic SD", produced by San-Ei Gen F.F.I., Inc.) and 0.05% by weight of decaglycerol monooleate and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate (product name "ML-750", produced by Sakamoto Yakuhin Kogyo Co., Ltd.) to be cooled rapidly, followed by suction filtration and vacuum drying, whereby a particulate composition was obtained. The solid fat content of tripalmitin and the results of the intestinal disintegration test of the particulate composition obtained were shown in Table 1.

### (Comparative Example 2)

The preparation of a particulate composition was attempted in the same manner as in Example 1 except that a material prepared by heat-mixing 50 g of fully hardened rapeseed oil (produced by Yokozeki Oil & Fat Industries Co., Ltd.) and 50 g of medium chain fatty acid triglyceride (product name "Actor M2", produced by Riken Vitamin Co., Ltd.) was used as a fat composition. However, even though the S/O/W emulsion was added to an aqueous solution of gum arabic and decaglycerol monooleate cooled to 5°C, the oil phase part did not solidify, so that any desired particulate composition could not be obtained. The solid fat content of the fat composition used was shown in Table 1.

**[Table 1]**

| | Solid fat content at each temperature of fat composition used (%) | | Release rate of hydrophilic substance in intestinal disintegration test (%) |
|---|---|---|---|
| | 25°C | 37°C | |
| Example 1 | 93.2 | 88.2 | 22.5 |
| Example 2 | >67.9 | 67.9 | 56.9 |
| Example 3 | >70.2 | 70.2 | 42.4 |
| Example 4 | 81.6 | 73.7 | 45.1 |
| Example 5 | 60.2 | 53.1 | 80.2 |
| Example 6 | 88.3 | 84.4 | 62.0 |
| Example 7 | 88.0 | 83.5 | 77.5 |
| Example 8 | 87.6 | 83.2 | 52.8 |
| Example 9 | 88.0 | 83.7 | 50.0 |
| Example 10 | 87.7 | 83.1 | 88.7 |
| Example 11 | 80.5 | 71.7 | 94.2 |
| Example 12 | 87.4 | 83.1 | 66.5 |
| Example 14 | >66.4 | 66.4 | 52.4 |
| Example 16 | >87.8 | 87.8 | 40.6 |
| Comparative Example 1 | 99.6 | 99.3 | 14.5 |
| Comparative Example 2 | 45.5 | 38.8 | |

Table 1 clearly shows that the particulate compositions of the examples in which a fat composition having a solid fat content of 50% or more and 90% or less was used as a matrix were fine particulate compositions in which a hydrophilic substance was encapsulated in a high content and were particulate compositions exhibiting good enteric properties indicated by a release rate of a hydrophilic substance in a simulated intestinal fluid of 20% or more.

### (Second present invention)

### <Analysis of constituent fatty acid composition>

Fifty milligrams of a fat to be analyzed was dissolved in 5 ml of isooctane, then 1 ml of a 0.2 mol/L sodium methylate/methanol solution was added, and then a reaction was performed at 70°C for 15 minutes, whereby constituent fatty acids in the fat were methylesterificated. After the reaction liquid was neutralized with acetic acid, an appropriate amount of water was added, and the fatty acid methyl esters in the resulting organic phase were detected by gas chromatograph (model specification: 6890N, manufactured by Agilent), whereby the constituent fatty acid composition in the analyzed fat was analyzed.

### <Measurement of contents of triglycerides>

The composition of each of the triglycerides contained in the fat to be analyzed and its content were determined from retention times and peak area ratios of a chart produced by analysis with gas chromatograph equipped with a flame ionization detector and fitted with a capillary column. The measurement conditions are as follows:
Column: TAP-CB (manufactured by GL Sciences Inc.), 0.2 mm in inner diameter, 25 m in length
Temperature conditions: onset temperature was 100°C; after temperature elevation up to 320°C at a rate of temperature elevation of 10°C/minute, the temperature was held at 320°C for 8 minutes.

### <Disintegration test method>

In the same procedures as described above, release rates of a hydrophilic substance after treatments at 37°C for 20 minutes, 40 minutes, 1 hour, and 3 hours were evaluated.

### (Example 18)

Five hundred grams of medium chain fatty acid triglyceride (product name "Actor M2", produced by Riken Vitamin Co., Ltd.) and 500 g of fully hardened rapeseed oil (produced by Taiyo Yushi Corporation) were heat-mixed at 80°C. Ten grams of fixed lipase (product name "Lipozime TL IM", produced by Novozymes) was added thereto and a transesterification reaction was performed for 24 hours under stirring at 80°C. After the completion of the reaction, the fixed lipase was removed by suction filtration at 80°C using filter paper (produced by Advantec Toyo Kaisha, Ltd.; No.1), whereby a transesterified fat was obtained. Nine hundred grams of n-hexane was added to 300 g of the resulting transesterified fat and heated to 45°C, whereby the transesterified fat was dissolved completely. Then, a solution of the transesterified fat in hexane was cooled at a rate of 0.5°C/minute to 20°C under stirring. After holding at 20°C for 40 minutes, it was subjected to suction filtration and thereby a crystalline fraction was removed. The resulting filtrate fraction was heated to 35°C, held for 10 minutes, and cooled at a rate of 0.5°C/minute to 0°C under stirring. After holding at 0°C for 30 minutes, the crystalline fraction obtained was collected by suction filtration and hexane was evaporated with an evaporator, followed by steam distillation at 210°C, whereby a medium melting fraction was obtained. The triglyceride composition of the resulting medium melting fraction, the contents of the respective components, and the proportion of saturated fatty acids having 14 or more carbon atoms are shown in Table 2.

An S/O type microcapsule containing the medium melting fraction of the transesterified fat as a matrix was prepared as follows. To an oily component composed of 20 g of a medium melting fraction having been melted by being heated to a temperature of 35°C in advance and 1.0 g of tetraglycerol condensed ricinoleate (product name "POEM PR-100", produced by Riken Vitamin Co., Ltd.), 10 mL of a 10% by weight Food Red No.102 aqueous solution was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 35°C for 30 minutes under a reduced pressure condition of 13 kPa, whereby an S/O suspension was obtained. The S/O suspension was added to 200 mL of an aqueous solution having been heated to 70°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule was obtained. A disintegration test was performed using the microcapsule. The results are shown in Table 3.

### (Example 19)

To 900 g of fully hardened rapeseed oil (produced by Taiyo Yushi Corporation), 400 g of lauric acid (produced by Wako Pure Chemical Industries, Ltd.) and 2600 g of hexane (produced by Wako Pure Chemical Industries, Ltd.) were added and heated to 55°C. Fifteen grams of fixed 1,3-position specific lipase (product name "Lipozyme RMIM", produced by Novozymes) was added thereto and a transesterification reaction was performed for 8 hours under stirring at 55°C. After the completion of the reaction, the fixed lipase was removed by suction filtration at 80°C using filter paper (produced by Advantec Toyo Kaisha, Ltd.; No.1), and then hexane and a fatty acid were removed by using a thin film distillation instrument (manufactured by Sibata Scientific Technology Ltd.) at a flow rate of 100 mL/hour, a distillation temperature of 200°C, and a degree of vacuum of 6.7 Pa, whereby a transesterified fat was obtained. The resulting transesterified fat was heated to 70°C and then cooled to 46°C under stirring. After holding at 46°C for 300 minutes, it was subjected to suction filtration to remove a crystalline fraction, and thereby a fractionated fat was obtained. The triglyceride composition of the resulting fractionated fat, the contents of the respective components, and the proportion of saturated fatty acids having 14 or more carbon atoms are shown in Table 2.

An S/O type microcapsule containing the fractionated fat of the transesterified fat as a matrix was prepared as follows. To an oily component composed of 20 g of a fractionated fat having been melted by being heated to a temperature of 40°C in advance and 1.0 g of tetraglycerol condensed ricinoleate (product name "POEM PR-100", produced by Riken Vitamin Co., Ltd.), 10 mL of a 25% anserine aqueous solution was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 70°C for 30 minutes under a reduced pressure condition of 13 kPa, whereby an S/O suspension was obtained. The S/O suspension was added to 200 mL of an aqueous solution having been heated to 40°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule enclosing anserine was obtained.

### (Example 20)

To 450 g of fully hardened rapeseed oil (produced by Taiyo Yushi Corporation), 500 g of lauric acid (produced by Wako Pure Chemical Industries, Ltd.) and 1900 g of hexane (produced by Wako Pure Chemical Industries, Ltd.) were added and heated to 55°C. Fifteen grams of fixed 1,3-potion specific lipase (product name "Lipozyme RMIM", produced by Novozymes) was added thereto and a transesterification reaction was performed for 8 hours under stirring at 55°C. After the completion of the reaction, the fixed lipase was removed by suction filtration at 80°C using filter paper (produced by Advantec Toyo Kaisha, Ltd.; No.1), and then hexane and a fatty acid were removed by using a thin film distillation instrument (manufactured by Sibata Scientific Technology Ltd.) at a flow rate of 100 mL/hour, a distillation temperature of 200°C, and a degree of vacuum of 6.7 Pa, whereby a transesterified fat was obtained. The triglyceride composition of the resulting transesterified fat, the contents of the respective components, and the proportion of saturated fatty acids having 14 or more carbon atoms are shown in Table 2.

An S/O type microcapsule containing the transesterified fat as a matrix was prepared as follows. To an oily component composed of 20 g of a fractionated fat having been melted by being heated to a temperature of 40°C in advance and 1.0 g of tetraglycerol condensed ricinoleate (product name "POEM PR-100", produced by Riken Vitamin Co., Ltd.), 10 mL of a 25% anserine aqueous solution was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 40°C for 30 minutes under a reduced pressure condition of 13 kPa, whereby an S/O suspension was obtained. The S/O suspension was added to 200 mL of an aqueous solution having been heated to 40°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule enclosing anserine was obtained.

### (Example 21)

To 900 g of tripalmitin (produced by Wako Pure Chemical Industries, Ltd.), 400 g of lauric acid (produced by Wako Pure Chemical Industries, Ltd.) and 2400 g of hexane (produced by Wako Pure Chemical Industries, Ltd.) were added and heated to 55°C. Thirteen grams of fixed 1,3-position specific lipase (product name "Lipozyme RMIM", produced by Novozymes) was added thereto and a transesterification reaction was performed for 8 hours under stirring at 55°C. After the completion of the reaction, the fixed lipase was removed by suction filtration at 80°C using filter paper (produced by Advantec Toyo Kaisha, Ltd.; No.1), and then hexane and a fatty acid were removed by using a thin film distillation instrument (manufactured by Sibata Scientific Technology Ltd.) at a flow rate of 100 mL/hour, a distillation temperature of 200°C, and a degree of vacuum of 6.7 Pa, whereby a transesterified fat was obtained. Nine hundred grams of n-hexane was added to 300 g of the resulting transesterified fat and heated to 45°C, whereby the transesterified fat was dissolved completely. Then, a solution of the transesterified fat in hexane was cooled at a rate of 0.5°C/minute to 20°C under stirring. After holding at 20°C for 40 minutes, it was subjected to suction filtration and thereby a crystalline fraction was removed. The resulting filtrate fraction was heated to 35°C, held for 10 minutes, and cooled at a rate of 0.5°C/minute to 0°C under stirring. After holding at 0°C for 30 minutes, the crystalline fraction obtained was collected by suction filtration and hexane was evaporated with an evaporator, followed by steam distillation at 210°C, whereby a medium melting fraction was obtained. The triglyceride composition of the resulting medium melting fraction, the contents of the respective components, and the proportion of saturated fatty acids having 14 or more carbon atoms are shown in Table 2.

An S/O type microcapsule containing the medium melting fraction of the transesterified fat as a matrix was prepared as follows. To an oily component composed of 20 g of a medium melting fraction having been melted by being heated to a temperature of 40°C in advance and 1.0 g of sucrose erucate (product name "ER-290", produced by Mitsubishi-Kagaku Foods Corporation), 1 g of lactoferrin was added and dispersed with a homogenizer (T.K. Homomixer MARK II20, manufactured by PRIMIX Corporation), whereby an S/O suspension was obtained. The S/O suspension was fed to a double-fluid nozzle (hollow cone spray nozzle, manufactured by Ikeuchi Co., Ltd.) at a pressure of 0.3 MPa with a pump and sprayed into a cooling field of 10°C, whereby an S/O type microcapsule enclosing lactoferrin was obtained.

### (Example 22)

To 900 g of tripalmitin, 400 g of capric acid (produced by Wako Pure Chemical Industries, Ltd.) and 2400 g of hexane (produced by Wako Pure Chemical Industries, Ltd.) were added and heated to 55°C. Thirteen grams of fixed lipase (product name "Lipozyme RMIM", produced by Novozymes) was added thereto and a transesterification reaction was performed for 8 hours under stirring at 55°C. After the completion of the reaction, the fixed lipase was removed by suction filtration at 80°C using filter paper (produced by Advantec Toyo Kaisha, Ltd.; No.1). Thereafter, hexane and a fatty acid were removed by using a thin film distillation instrument (manufactured by Sibata Scientific Technology Ltd.) at a flow rate of 100 mL/hour, a distillation temperature of 200°C, and a degree of vacuum of 6.7 Pa. Nine hundred grams of n-hexane was added to 300 g of the resulting transesterified fat and heated to 45°C, whereby the transesterified fat was dissolved completely. A solution of the transesterified fat in hexane was cooled at a rate of 0.5°C/minute to 20°C under stirring. After holding at 20°C for 40 minutes, it was subjected to suction filtration and thereby a crystalline fraction was removed. The resulting filtrate fraction was heated to 35°C, held for 10 minutes, and cooled at a rate of 0.5°C/minute to 0°C under stirring. After holding at 0°C for 30 minutes, the crystalline fraction obtained was collected by suction filtration and hexane was evaporated with an evaporator, followed by steam distillation at 210°C, whereby a medium melting fraction was obtained. The triglyceride composition of the resulting medium melting fraction, the contents of the respective components, and the proportion of saturated fatty acids having 14 or more carbon atoms are shown in Table 2.

An S/O type microcapsule containing the medium melting fraction of the transesterified fat as a matrix was prepared as follows. To an oily component composed of 20 g of a fractionated fat having been melted by being heated to a temperature of 40°C in advance and 1.0 g of sucrose erucate (product name "ER-290", produced by Mitsubishi-Kagaku Foods Corporation), 1 g of lactoferrin was added and dispersed with a homogenizer (T.K. Homomixer MARK II20, manufactured by PRIMIX Corporation), whereby an S/O suspension was obtained. The S/O suspension was fed to a double-fluid nozzle (hollow cone spray nozzle, manufactured by Ikeuchi Co., Ltd.) at a pressure of 0.3 MPa with a pump and sprayed into a cooling field of 10°C, whereby an S/O type microcapsule enclosing lactoferrin was obtained.

### (Example 23)

Five hundred grams of medium chain fatty acid triglyceride (product name "Actor M2", produced by Riken Vitamin Co., Ltd.) and 500 g of fully hardened rapeseed oil (produced by Taiyo Yushi Corporation) were mixed, and the mixture was subjected to heat-dehydration at 90°C and a degree of vacuum of 4.0 kPa, and then 1 g of sodium methylate was added thereto, followed by a transesterification reaction at 90°C and a degree of vacuum of 4.0 kPa for 20 minutes. After the reaction, the reaction was terminated by the addition of enough water and at the same time a soap content generated was removed. Then, heat-dehydration was performed at 90°C and a degree of vacuum of 4.0 kPa, 20 g of white clay (produced by Mizusawa Industrial Chemicals, Ltd.) was added and held for 10 minutes, and then the white clay was removed by suction filtration using filter paper (produced by Advantec Toyo Kaisha, Ltd.; No.1), whereby a transesterified fat was obtained. Nine hundred grams of n-hexane was added to 300 g of the resulting transesterified fat and heated to 45°C, whereby the transesterified fat was dissolved completely. A solution of the transesterified fat in hexane was cooled at a rate of 0.5°C/minute to 20°C under stirring. After holding at 20°C for 40 minutes, it was subjected to suction filtration and thereby a crystalline fraction was removed. The resulting filtrate fraction was heated to 35°C, held for 10 minutes, and cooled at a rate of 0.5°C/minute to 0°C under stirring. After holding at 0°C for 30 minutes, the crystalline fraction obtained was collected by suction filtration and hexane was evaporated with an evaporator, followed by steam distillation at 210°C, whereby a medium melting fraction was obtained. The triglyceride composition of the resulting medium melting fraction, the contents of the respective components, and the proportion of saturated fatty acids having 14 or more carbon atoms are shown in Table 2.

An S/O type microcapsule containing the medium melting fraction of the transesterified fat as a matrix was prepared as follows. To an oily component composed of 20 g of a fractionated fat having been melted by being heated to a temperature of 60°C in advance and 1.0 g of tetraglycerol condensed ricinoleate (product name "POEM PR-100", produced by Riken Vitamin Co., Ltd.), 10 mL of a 30% glutathione aqueous solution was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 35°C for 30 minutes under a reduced pressure condition of 13 kPa, whereby an S/O suspension was obtained. The S/O suspension was added to 200 mL of an aqueous solution having been heated to 35°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule enclosing glutathione was obtained.

### (Example 24)

To 900 g of fully hardened rapeseed oil (produced by Taiyo Yushi Corporation), 400 g of capric acid (produced by Wako Pure Chemical Industries, Ltd.) and 2400 g of hexane (produced by Wako Pure Chemical Industries, Ltd.) were added and heated to 55°C. Thirteen grams of fixed lipase (product name "Lipozyme RMIM", produced by Novozymes) was added thereto and a transesterification reaction was performed for 8 hours under stirring at 55°C. After the completion of the reaction, the fixed lipase was removed by suction filtration at 80°C using filter paper (produced by Advantec Toyo Kaisha, Ltd.; No. 1) Then, hexane and a fatty acid were removed by using a thin film distillation instrument (manufactured by Sibata Scientific Technology Ltd.) at a flow rate of 100 mL/hour, a distillation temperature of 200°C, and a degree of vacuum of 6.7 Pa, whereby a transesterified fat was obtained. Nine hundred grams of n-hexane was added to 300 g of the resulting transesterified fat and heated to 45°C, whereby the transesterified fat was dissolved completely. Then, a solution of the transesterified fat in hexane was cooled at a rate of 0.5°C/minute to 20°C under stirring. After holding at 20°C for 40 minutes, it was subjected to suction filtration and thereby a crystalline fraction was removed. The resulting filtrate fraction was heated to 35°C, held for 10 minutes, and cooled at a rate of 0.5°C/minute to 0°C under stirring. After holding at 0°C for 30 minutes, the crystalline fraction obtained was collected by suction filtration and hexane was evaporated with an evaporator, followed by steam distillation at 210°C, whereby a medium melting fraction was obtained. The triglyceride composition of the resulting medium melting fraction, the contents of the respective components, and the proportion of saturated fatty acids having 14 or more carbon atoms are shown in Table 2.

An S/O type microcapsule containing the medium melting fraction of the transesterified fat as a matrix was prepared as follows. To an oily component composed of 20 g of a fractionated fat having been melted by being heated to a temperature of 40°C in advance and 1.0 g of tetraglycerol condensed ricinoleate (product name "POEM PR-100", produced by Riken Vitamin Co., Ltd.), 10 mL of a 30% glutathione aqueous solution was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 40°C for 30 minutes under a reduced pressure condition of 13 kPa, whereby an S/O suspension was obtained. The S/O suspension was added to 200 mL of an aqueous solution having been heated to 40°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule enclosing glutathione was obtained.

### (Example 25)

To 450 g of fully hardened rapeseed oil (produced by Taiyo Yushi Corporation), 500 g of lauric acid (produced by Wako Pure Chemical Industries, Ltd.) and 1900 g of hexane (produced by Wako Pure Chemical Industries, Ltd.) were added and heated to 55°C. Fifteen grams of fixed 1,3-position specific lipase (product name "Lipozyme RMIM", produced by Novozymes)) was added thereto and a transesterification reaction was performed for 8 hours under stirring at 55°C. After the completion of the reaction, the fixed lipase was removed by suction filtration at 80°C using filter paper (produced by Advantec Toyo Kaisha, Ltd.; No.1). Then, hexane and a fatty acid were removed by using a thin film distillation instrument (manufactured by Sibata Scientific Technology Ltd.) at a flow rate of 100 mL/hour, a distillation temperature of 200°C, and a degree of vacuum of 6.7 Pa, whereby a transesterified fat was obtained. To 20 g of the resulting transesterified fat was added 10 g of medium chain fatty acid triglyceride (product name "Actor M2", produced by Riken Vitamin Co., Ltd.) at 40°C, followed by mixing, whereby a mixed fat was prepared. The triglyceride composition of the resulting mixed fat, the contents of the respective components, and the proportion of saturated fatty acids having 14 or more carbon atoms are shown in Table 2.

An S/O type microcapsule containing the mixed fat composed of the transesterified fat and the medium chain fatty acid triglyceride as a matrix was prepared as follows.

To an oily component composed of the above-described mixed fat having been melted by being heated to a temperature of 40°C in advance and 1.0 g of tetraglycerol condensed ricinoleate (product name "POEM PR-100", produced by Riken Vitamin Co., Ltd.), 10 mL of an aqueous solution containing 10% by weight of Food Red No.102 was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 40°C for 30 minutes under a reduced pressure condition of 13 kPa, whereby an S/O suspension was obtained. The S/O suspension was added to 200 mL of an aqueous solution having been heated to 40°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule was obtained.

### (Example 26)

To 300 g of medium chain fatty acid triglyceride (product name "Actor M2", produced by Riken Vitamin Co., Ltd.), 700 g of stearic acid (produced by Wako Pure Chemical Industries, Ltd.) and 2000 g of hexane (produced by Wako Pure Chemical Industries, Ltd.) were added and heated to 55°C. Thirteen grams of fixed 1,3-position specific lipase (product name "Lipozyme RMIM", produced by Novozymes) was added thereto and a transesterification reaction was performed for 8 hours under stirring at 55°C. After the completion of the reaction, the fixed lipase was removed by suction filtration at 80°C using filter paper (produced by Advantec Toyo Kaisha, Ltd.; No.1). Thereafter, hexane and a fatty acid were removed by using a thin film distillation instrument (manufactured by Sibata Scientific Technology Ltd.) at a flow rate of 100 mL/hour, a distillation temperature of 200°C, and a degree of vacuum of 6.7 Pa. Nine hundred grams of n-hexane was added to 300 g of the resulting transesterified fat and heated to 45°C, whereby the transesterified fat was dissolved completely. Then, a solution of the transesterified fat in hexane was cooled at a rate of 0.5°C/minute to 20°C under stirring. After holding at 20°C for 40 minutes, it was subjected to suction filtration and thereby a crystalline fraction was removed. The resulting filtrate fraction was heated to 35°C, held for 10 minutes, and cooled at a rate of 0.5°C/minute to 0°C under stirring. After holding at 0°C for 30 minutes, the crystalline fraction obtained was collected by suction filtration and hexane was evaporated with an evaporator, followed by steam distillation at 210°C, whereby a medium melting fraction was obtained. The triglyceride composition of the resulting medium melting fraction, the contents of the respective components, and the proportion of saturated fatty acids having 14 or more carbon atoms are shown in Table 2. An S/O type microcapsule containing the medium melting fraction of the transesterified fat as a matrix was prepared as follows. To an oily component composed of 20 g of a medium melting fraction having been melted by being heated to a temperature of 50°C in advance and 1.0 g of tetraglycerol condensed ricinoleate (product name "POEM PR-100", produced by Riken Vitamin Co., Ltd.), 10 mL of an aqueous solution containing 10% by weight of Food Red No.102 was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 50°C for 30 minutes under a reduced pressure condition of 13 kPa, whereby an S/O suspension was obtained. The S/O suspension was added to 200 mL of an aqueous solution having been heated to 50°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule was obtained. A disintegration test was performed using the microcapsule. The results are shown in Table 3.

### (Example 27)

To 300 g of medium chain fatty acid triglyceride (product name "Actor M2", produced by Riken Vitamin Co., Ltd.), 700 g of stearic acid (produced by Wako Pure Chemical Industries, Ltd.) and 2000 g of hexane (produced by Wako Pure Chemical Industries, Ltd.) were added and heated to 55°C. Thirteen grams of fixed 1,3-position specific lipase (product name "Lipozyme RMIM", produced by Novozymes) was added thereto and a transesterification reaction was performed for 8 hours under stirring at 55°C. After the completion of the reaction, the fixed lipase was removed by suction filtration at 80°C using filter paper (produced by Advantec Toyo Kaisha, Ltd.; No.1). Thereafter, hexane and a fatty acid were removed by using a thin film distillation instrument (manufactured by Sibata Scientific Technology Ltd.) at a flow rate of 100 mL/hour, a distillation temperature of 200°C, and a degree of vacuum of 6.7 Pa. Nine hundred grams of n-hexane was added to 300 g of the resulting transesterified fat and heated to 45°C, whereby the transesterified fat was dissolved completely. Then, a solution of the transesterified fat in hexane was cooled at a rate of 0.5°C/minute to 20°C under stirring. After holding at 20°C for 40 minutes, it was subjected to suction filtration and thereby a crystalline fraction was removed. The resulting filtrate fraction was heated to 35°C, held for 10 minutes, and cooled at a rate of 0.5°C/minute to 0°C under stirring. After holding at 0°C for 30 minutes, the crystalline fraction obtained was collected by suction filtration and hexane was evaporated with an evaporator, followed by steam distillation at 210°C, whereby a medium melting fraction was obtained. To 20 g of the medium melting fraction was added 2.0 g of medium chain fatty acid triglyceride (product name "Actor M2", produced by Riken Vitamin Co., Ltd.) at 45°C as liquid oil, followed by mixing, whereby a mixed fat was prepared. The triglyceride composition of the resulting mixed fat, the contents of the respective components, and the proportion of saturated fatty acids having 14 or more carbon atoms are shown in Table 2.

An S/O type microcapsule containing the mixed fat composed of the medium melting fraction of the transesterified fat and the liquid oil as a matrix was prepared as follows. To an oily component composed of the above-described mixed fat having been melted by being heated to a temperature of 45°C in advance and 1.0 g of tetraglycerol condensed ricinoleate (product name "POEM PR-100", produced by Riken Vitamin Co., Ltd.), 10 mL of an aqueous solution containing 10% by weight of Food Red No.102 was added, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 45°C for 30 minutes under a reduced pressure condition of 13 kPa, whereby an S/O suspension was obtained. The S/O suspension was added to 200 mL of an aqueous solution having been heated to 45°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule was obtained. A disintegration test was performed using the microcapsule. The results are shown in Table 3.

### (Example 28)

Fifty grams of crystalline cellulose for tablet coating, 0.5 g of magnesium, stearate, and 50 g of lactoferrin were mixed and then pressed into a tablet with a tablet presser (WPM-5, manufactured by Okada Seiko Co., Ltd.), using a pounder with a diameter of 12.0 mm at a tabletting pressure of 0.5 ton/cm², whereby a tablet was obtained. Then, the medium melting fraction of the transesterified fat produced in Example 26 was heated to 55°C to be melted, thereby forming a coating fat. While the prepared tablet was rotated, the coating fat was sprayed through a single-fluid nozzle (hollow cone spray nozzle, manufactured by Ikeuchi Co., Ltd.) to apply a coating treatment to the surface of the tablet, whereby a coated tablet having a coating fat layer with a thickness of about 0.5 mm on the tablet surface was obtained.

### (Comparative Example 3)

To an oily component composed of 20 g of tripalmitin (produced by Wako Pure Chemical Industries, Ltd., tripalmitin acid content 98.2% by weight) having been melted by being heated to a temperature of 80°C in advance and 1.0 g of tetraglycerol condensed ricinoleate (product name "POEM PR-100", produced by Riken Vitamin Co., Ltd.), 10 mL of an aqueous solution containing 10% by weight of Food Red No.102 was added, followed by performing emulsification and dispersion with a homogenizer, whereby a W/O emulsion was prepared. Subsequently, the W/O emulsion was subjected to moisture removal while being stirred at a temperature of 80°C for 20 minutes under a reduced pressure condition of 13 kPa, whereby an S/O suspension was obtained. An S/O type microcapsule was obtained in the same manner as in Example 18 except that the S/O suspension was added to 300 mL of an aqueous solution having been heated to 70°C in advance and containing 0.5% by weight of gum arabic and 0.05% by weight of decaglycerol monooleate and stirred with a disk turbine blade, whereby an S/O/W emulsion was prepared. A disintegration test was performed using the microcapsule. The results are shown in Table 3.

### (Comparative Example 4)

To 20 g of medium chain fatty acid triglyceride (product name "Actor M2", produced by Riken Vitamin Co., Ltd.) and 1.0 g of tetraglycerol condensed ricinoleate (product name "POEM PR-100", produced by Riken Vitamin Co., Ltd.), 30 g of the fat of Example 20 whose temperature had been adjusted to 45°C was mixed, whereby an oily component was prepared. The triglyceride composition of the resulting oily component, the contents of the respective components, and the proportion of saturated fatty acids having 14 or more carbon atoms are shown in Table 2.

To the above-mentioned oily component, 10 mL of a 10% by weight Food Red No.102 aqueous solution was added, the temperature thereof was adjusted to 35°C, followed by performing emulsification and dispersion with a homogenizer (T.K. Homomixer MARK II20, manufactured by PRIMIX Corporation), whereby a W/O emulsion was prepared. Subsequently, the W/O emulsion was subjected to moisture removal while being stirred at a temperature of 70°C for 30 minutes under a reduced pressure condition of 13 kPa, whereby an S/O suspension was obtained. The S/O suspension was added to 200 mL of an aqueous solution having been heated to 45°C in advance and containing 0.5% by weight of gum arabic (product name "Gum Arabic SD", produced by San-Ei Gen F.F.I., Inc.) and 0.03% by weight of decaglycerol monooleate (product name "POEM J-0381V", produced by Riken Vitamin Co., Ltd.) and stirred with a disk turbine blade, whereby an S/O/W emulsion was prepared. Thereafter, the S/O/W emulsion was added in one portion to 400 mL of an aqueous solution having been cooled to 5°C in advance and containing 0.5% by weight of gum arabic and 0.03% by weight of decaglycerol monooleate to be cooled rapidly, followed by suction filtration and vacuum drying, whereby an S/O type microcapsule was obtained. The resulting microcapsule was not capable of being handled because it was very soft and sticky and particles thereof stuck together.

**[Table 2]**

| | Solid fat content at each temperature of fat composition used (%) | | Triglyceride compositions* of fat compositions of respective examples and comparative example, and their contents (% by weight) | | | | | | | | | | | | Proportion of saturrated fatty acid having 14 or more carbon atoms (% by weight) |
|---|---|---|---|---|---|---|---|---|---|---|---|---|---|---|---|
| | | | Triglyceride (A) containing saturated fatty acid having 6 to 12 carbon atoms and saturated fatty acid having 14 or more carbon atoms | | | | | | | | | | | Other than (A) | |
| | 25°C | 37°C | C8:C8:C18 | C8:C18:C18 | C10:C10:C16 | C10:C10:C18 | C10:C16:C16 | C10:C18:C18 | C12:C12:C16 | C12:C16:C16 | C12:C12:C18 | C12:C18C18 | Total | | |
| Example 18 | 87.8 | 25 | 7.5 | 72.2 | | | | | | | | | 79.7 | 20.3 | 66 |
| Example 19 | 96.4 | 76.8 | | | | | | | | | 19.6 | 43.4 | 63 | 37.0 | 61 |
| Example 20 | 95.2 | 74.8 | | | | | | | | | 34.0 | 38.9 | 72.9 | 27.1 | 76 |
| Example 21 | 97.6 | 89.9 | | | | | | | 21.3 | 53.5 | | | 74.8 | 25.2 | 60 |
| Example 22 | 96.9 | 88.1 | | | 8.2 | | 45.0 | | | | | | 53.2 | 46.8 | 79 |
| Example 23 | 86.5 | 55.8 | 6.2 | 69.3 | | | | | | | | | 75.5 | 24.5 | 67 |
| Example 24 | 89 | 65.2 | | | | 7.2 | | 68.2 | | | | | 75.4 | 24.6 | 67 |
| Example 25 | 62.2 | 61.1 | | | | | | | | | 22.8 | 26.1 | 48.9 | 51.1 | 51 |
| Example 26 | 94 | 88.3 | 7.2 | 85.2 | | | | | | | | | 92.4 | 7.6 | 65 |
| Example 27 | 84 | 78.3 | 6.3 | 74.0 | | | | | | | | | 80.3 | 19.7 | 56 |
| Example 28 | 94 | 88.3 | 7.2 | 85.2 | | | | | | | | | 92.4 | 7.6 | 65 |
| Comparative Example 4 | 57.1 | 44.9 | | | | | | | | | 20.0 | 23.5 | 43.5 | 56.5 | 46 |

**[Table 3]**

| Time (min) | Release rate of enclosed substance (%) | | | |
|---|---|---|---|---|
| | Example 18 | Example 26 | Example 27 | Comparative Example 3 |
| 0 | 0 | 0 | 0 | 0 |
| 20 | 26.2 | 7.6 | 21.5 | 3.3 |
| 40 | 53.2 | 16.2 | 50.9 | 7.2 |
| 60 | 72.3 | 26.9 | 67.8 | 14.5 |
| 360 | 100 | 96.4 | 100 | 100 |

The above-mentioned results show that good enteric properties are exhibited by S/O type microcapsule formulations using, as matrix components, fat compositions containing 45% by weight or more of a triglyceride including at least both a saturated fatty acid having 6 to 12 carbon atoms and a saturated fatty acid having 14 or more carbon atoms as constituent fatty acids, wherein the proportion of the saturated fatty acid having 14 or more carbon atoms in the constituent fatty acids of the whole fat exceeds 50% by weight.

## Claims

1. A particulate composition, wherein a hydrophilic substance is polydispersed in a matrix of a fat composition having a solid fat content at 25°C of 58% or more and a solid fat content at 37°C of 90% or less.

2. The particulate composition according to claim 1, wherein the solid fat content at 37°C of the fat composition is 50% or more.

3. The particulate composition according to claim 1 or 2, wherein the fat composition is a coating fat composition containing 45% by weight or more of a triglyceride comprising at least both a saturated fatty acid having 6 to 12 carbon atoms and a saturated fatty acid having 14 or more carbon atoms as constituent fatty acids and the proportion of the saturated fatty acid having 14 or more carbon atoms in the constituent fatty acids of the whole fat composition exceeds 50% by weight.

4. The particulate composition according to claim 3, wherein in the coating fat composition, the content of a triglyceride comprising at least both a saturated fatty acid having 8 to 12 carbon atoms and a saturated fatty acid having 16 or 18 carbon atoms as constituent fatty acids is 50 to 90% by weight.

5. The particulate composition according to any one of claims 1 to 4, wherein the fat composition is a mixture of 95 to 60% by weight of a high-melting-point fat and 5 to 40% by weight of a low-melting-point oil.

6. The particulate composition according to claim 5, wherein the high-melting-point fat is at least one member selected from the group consisting of fractionated palm oil, hardened palm oil, fully hardened palm oil, hardened rapeseed oil, fully hardened rapeseed oil, tristearin, and tripalmitin.

7. The particulate composition according to claim 5 or 6, wherein the low-melting-point oil is at least one member selected from the group consisting of palm kernel oil, coconut oil, medium chain fatty acid triglyceride, soybean oil, rice oil, corn oil, olive oil, rapeseed oil, sunflower oil, perilla oil, low-melting fractions of fish oil, diglyceride, and oleic acid.

8. The particulate composition according to any one of claims 1 to 7, wherein the fat composition is obtained by a transesterification reaction.

9. The particulate composition according to claim 8, wherein an enzyme or a microorganism is used in the transesterification reaction.

10. The particulate composition according to any one of claims 1 to 9, wherein the weight ratio of the hydrophilic substance to the fat composition is within the range of from 0.01/99.99 to 70/30.

11. The particulate composition according to any one of claims 1 to 10, further comprising a hydrophobic component.

12. The particulate composition according to any one of claims 1 to 11, wherein the release rate of the hydrophilic substance in an intestinal disintegration test is 40% or more.

13. A coating fat composition containing 45% by weight or more of a triglyceride comprising at least both a saturated fatty acid having 6 to 12 carbon atoms and a saturated fatty acid having 14 or more carbon atoms as constituent fatty acids, wherein the proportion of the saturated fatty acid having 14 or more carbon atoms in the constituent fatty acids of the whole fat composition exceeds 50% by weight.

14. The coating fat composition according to claim 13, wherein the fat composition is obtained by a transesterification reaction.
